# EUROPEAN PATENT APPLICATION

(11) **EP 4 026 588 A1**
(43) Date of publication of application: **13.07.2022**
(21) Application number: 20861322.4
(22) Date of filing: 04.09.2020
(51) Int. Cl.: A61P 35/00, G01N 21/65, C12N 5/09, C12Q 1/02, A61K 33/243, G01N 33/50, A61K 31/7068

(54) **METHOD FOR ACQUIRING DATA FOR DISTINGUISHING PRESENCE OF CANCER CELLS AND/OR DISTINGUISHING ANTICANCER DRUG RESISTANCE, METHOD FOR ACQUIRING PREDICTION DATA, USE OF DISTINCTION MARKER IN SAME, AND DISTINGUISHING KIT**

(30) Priority: 05.09.2019 JP 2019162162
(71) Applicant: Keio University, Tokyo, 108-8345 (JP)
(72) Inventor: HISHIKI, Takako, Tokyo 160-8582 (JP); SUEMATSU, Makoto, Tokyo 160-8582 (JP)
(74) Representative: Gill Jennings & Every LLP
(86) International application number: PCT/JP2020/033640
(87) International publication number: WO 2021/045202

(57) **Abstract**

The present invention aims to provide a method of acquiring data for determination of, and a method of acquiring prediction data on the presence of cancer cells and/or the resistance to anticancer drugs, use of a marker for determining thereof, and a kit for determining thereof, in particular, to determine the resistance to anticancer drugs before administration of the anticancer drugs to patients. The resistance of cancer tissues of cancer patients to anticancer drugs can be determined by detecting polysulfide, which is a marker for the resistance to anticancer drugs, in the cancer tissues of the cancer patients before administration of the anticancer drugs.

## Description

### TECHNICAL FIELD

The present invention relates to a method of acquiring data for determination of, and a method of acquiring prediction data on the presence of cancer cells and/or the resistance to an anticancer drug, use of a marker for determining thereof, and kit for determining thereof.

### BACKGROUND ART

Cancers generally produce various metabolites that are advantageous for their own survival due to their gene mutations and show metabolic characteristics different from the surrounding non-cancer tissues. It has been also found that cancers grow by actively exploiting metabolites required for their survival from the surrounding normal tissues. Among such metabolites, the involvement of sulfur-containing metabolites, such as glutathione, cysteine, and hydrogen sulfide in, for example, the survival, proliferation, and drug resistance of cancer cells as strong antioxidants has been suggested, but actually been unclear.

Accordingly, the present inventors have performed various analyses to obtain findings on survival, proliferation, and drug resistance of cancer cells, and have found in analysis using glioblastoma model mice that the glioblastoma portion in the disease model is rich in polysulfide (Non-patent Document 1). However, there has not been so far a human analysis on polysulfide, nor analyses in cancers other than glioblastoma.

In the case of ovarian cancer, the cancer is often already in advanced stages when it is found. The standard treatment for such advanced ovarian cancer includes ovariectomy, followed by chemotherapy, such as by anticancer drug, to kill ovarian cancer cells remaining in the body.

In common, anticancer drugs have strong adverse effects, and may cause nausea, general malaise, hair loss, rash, anemia, and others. In addition, anticancer drugs may cause serious adverse effects such as acute renal failure, hepatic dysfunction, pancytopenia, anaphylaxis, and thus they have not necessarily given only advantageous effect to all cancer patients. Furthermore, even after treatments that can cause such various adverse effects, recurrence of ovarian cancer occurs at a certain frequency, and thus some cancer patients have not received sufficient therapeutic effects.

Under these circumstances, predictable therapeutic effects of anticancer drugs before administration to cancer patients has been desired in order to select an appropriate anticancer drug for each patient for improved balance between the invasiveness of anticancer drug treatment to cancer patients and the therapeutic effects of the anticancer drug treatment.

### PRIOR ART REFERENCES

### NON-PATENT DOCUMENTS

Non-patent Document 1: Nature Communications, 2018, 9, Article number: 1561
Non-patent Document 2: Analyt Chem, 2006, 78, 2631-2639
Non-patent Document 3: Inorg. Chem., 1976, 15, 1759-1763
Non-patent Document 4: Phys Chem Inorb Phys., 2015, 17, 5155-5171

### SUMMARY OF THE INVENTION

### PROBLEMS TO BE SOLVED BY THE INVENTION

The present invention aims to provide a method of acquiring data for determination of, and a method of acquiring prediction data on the presence of cancer cells and/or the resistance to an anticancer drug, use of a marker for determining thereof, and a kit for determining thereof. The present invention in particular aims to determine the resistance to an anticancer drug before administration of the anticancer drug to patients.

### MEANS FOR SOLVING THE PROBLEMS

The present inventors have found that cancer tissues include those that are susceptible to anticancer drugs, and those that are insusceptible to anticancer drugs or have resistance to anticancer drugs. For example, cisplatin, when used as an anticancer drug, has shown to be less effective in ovarian clear cell carcinoma (CCC) (i.e., the CCC is resistant to the anticancer drug), but more effective in ovarian serous carcinoma (SC) (i.e., the SC is not resistant to the anticancer drug). The present inventors also have found that Raman spectroscopy on cancer tissues with anticancer drug resistance shows the presence of a specific peak in the spectrum of CCC. In addition, the specific peak found in the cancer tissues with anticancer drug resistant has been found at around a Raman shift of 480 cm⁻¹ (kayser) in Raman spectroscopy. It has been found that the specific peak indicates high expression of polysulfide in the cancer tissues.

In other words, cancer tissues with anticancer drug resistance highly express polysulfide that can be used as a marker for anticancer drug resistance. Thus, detection of the polysulfide that can be used as a marker for anticancer drug resistance in cancer patients allows for determination whether cancer tissues in the cancer patients are resistant to anticancer drugs. It is also possible to determine whether cancer tissues in the cancer patients are resistant to anticancer drugs even before administration of the anticancer drugs.

Accordingly, the present application provides the following aspects.
[1] A method of acquiring data for determining the presence of cancer cells and/or the resistance of the cancer cells to an anticancer drug in a target tissue, the method comprising a step of measuring the level of polysulfide in the target tissue,
   wherein the anticancer drug is reactive to the polysulfide.
[2] The method according to [1], wherein the measurement is performed before administration of the anticancer drug.
[3] The method according to [1] or [2], wherein the measurement step is performed by detecting a peak that is specific to the polysulfide by Raman spectroscopy.
[4] The method according to [3], wherein the specific peak appears at a Raman shift of 480 ± 10 cm⁻¹ when the Raman spectroscopy is performed on a cancer tissue.
[5] The method according any one of [1] to [4], wherein the anticancer drug is cisplatin or gemcitabine.
[6] The method according any one of [1] to [5], wherein the target tissue is an ovarian cancer tissue or a pancreas cancer tissue.
[7] The method according to [6], wherein the ovarian cancer tissue is an ovarian clear cell carcinoma tissue.
[8] A method of acquiring prediction data on the presence of cancer cells and/or the resistance of the cancer cells to an anticancer drug,
   wherein the method uses the level of polysulfide as an index,
   wherein when the level of polysulfide in a target tissue is higher than that in a control tissue, prediction is made that cancer cells are present and/or that the cancer cells have resistance to the anticancer drug in the target tissue,
   wherein the anticancer drug is reactive to the polysulfide.
[9] Use of polysulfide as a marker for determining the presence of cancer cells and/or the resistance of the cancer cells to an anticancer drug,
   wherein the anticancer drug is reactive to the polysulfide.
[10] A kit for determining the presence of cancer cells and/or the resistance of the cancer cells to an anticancer drug, the kit comprising a substrate for measuring the level of polysulfide by Raman spectroscopy,
   wherein the anticancer drug is reactive to the polysulfide.

### EFFECT OF THE INVENTION

The method of acquiring data for determination of, and the method of acquiring prediction data on the presence of cancer cells and/or the resistance to an anticancer drug, as well as the marker for determining thereof, and the kit for determining thereof according to the present invention enables determination of the resistance to the anticancer drug.

Based on the results from determination of anticancer drug resistance, a treatment plan can be established to determine whether to administer the anticancer drug or other treatment such as surgery.

### BRIEF DESCRIPTION OF THE DRAWINGS

FIG. 1 shows the average surface enhanced Raman spectra of tissue sections from ovarian clear cell carcinoma (CCC) or ovarian serous carcinoma (SC), and the spectrum representing the difference between the spectra (CCC-SC). The top and middle panels show the average surface enhanced Raman spectra of tissue sections from CCC (n = 12) and SC (n = 12), respectively. All tissue sections are collected from NCC Biobank.
FIG. 2 shows a graph comparing the SERS signal intensities, which are the heights at a Raman shift of 480 cm⁻¹, between CCC-derived tissue sections and SC-derived tissue sections. The unit of the intensity is arbitrary unit (a.u.). When the p value is less than 0.05 in false discovery rate (FDR) analysis, the difference is considered as statistically significant. SERS signals with Raman shifts within ±10 cm⁻¹ from the peak position are considered as the same signals.
FIG. 3 shows pictures of in vitro gel shift assay confirming that disturbance of double strand DNA supercoiling by cisplatin (CDDP) is attenuated by Na₂S, Na₂S₂, Na₂S₃, and Na₂S₄.
FIG. 4 shows a representative conventional resonance Raman spectrum of a 3 mM CDDP solution.
FIG. 5 shows that addition of CDDP reduces the signal intensity of the peak at a Raman shift of 471 cm⁻¹ for Na₂S₄ in a conventional resonance Raman spectrum depending on the concentration of the added CDDP.
FIG. 6 shows representative surface enhanced Raman spectra of 50 µM and 200 µM gemcitabine solutions.
FIG. 7 shows that addition of gemcitabine reduces the signal intensity of the peak at a Raman shift of 456 cm⁻¹ yielded by Na₂S₃ in a surface enhanced Raman spectrum depending on the concentration of the added gemcitabine.
FIG. 8 shows that addition of gemcitabine reduces the signal intensity of the peak at a Raman shift of 456 cm⁻¹ yielded by r Na₂S₄ in a surface enhanced Raman spectrum depending on the concentration of the added gemcitabine.

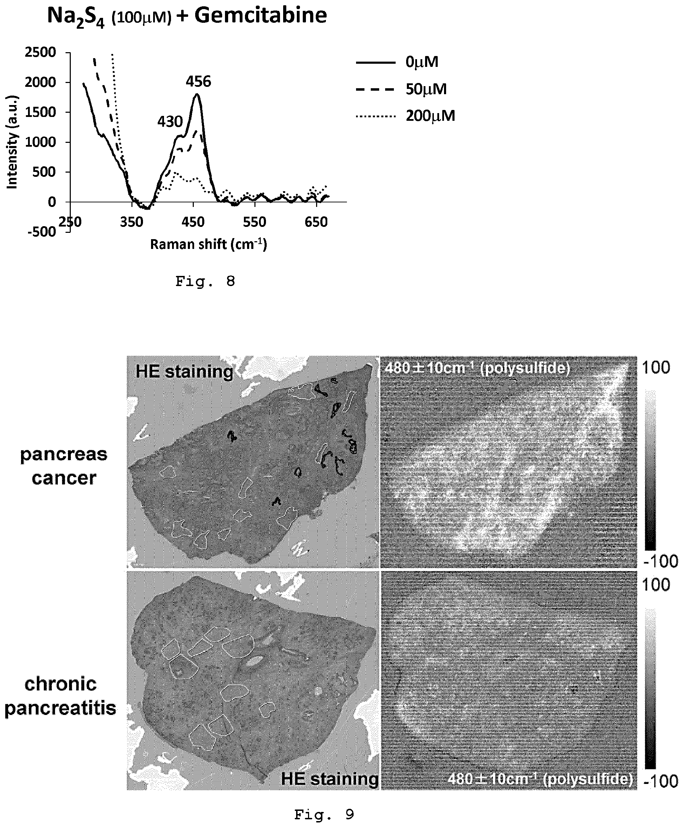

FIG. 9 shows visualization of polysulfide by SERS imaging of tissue sections obtained from pancreas cancer and chronic pancreatitis as a control. The left top panel is a photograph of HE staining of the pancreas cancer tissue section, while the right top panel is a photograph of SERS imaging at a Raman shift of 480 cm⁻¹ of the pancreas cancer tissue section. The left bottom panel is a photograph of HE staining of the chronic pancreatitis tissue section, while the right bottom panel is a photograph of SERS imaging at a Raman shift of 480 cm⁻¹ of the chronic pancreatitis tissue section. HE staining is performed using the sections after SERS imaging. In the HE staining, the black annotation represents cancerous portion, while the white annotation represents stromal portion.

### DETAILED DESCRIPTION OF THE INVENTION

An aspect of the present invention is a method of acquiring data for determining the presence of cancer cells and/or the resistance of the cancer cells to an anticancer drug in a target tissue, the method comprising a step of measuring the level of polysulfide in the target tissue, wherein the anticancer drug is reactive to the polysulfide.

Polysulfide in the present invention refers to a polymer compound or a salt thereof, containing one or more disulfide bond, which is represented by the chemical formula R-Sₓ-R. In the chemical formula above, R represents, for example but not limited to, H, Na, or an alkyl group optionally substituted with any substituent, and preferably Na. x represents any integer of 2 or more, and preferably an integer of 2 to 4.

Polysulfide in the present invention is not particularly limited, and is preferably Na₂S₂, Na₂S₃, or Na₂S₄ when R represents Na.

The method of measuring the level of polysulfide is not particularly limited as long as the level of polysulfide can be measured. For example, the level of polysulfide can be measured by Raman spectroscopy or electrochemical analysis as described in Non-patent Document 2, and is preferably measured by Raman spectroscopy. Several methods for Raman spectroscopy are known by those skilled in the art. For example, without limitation, conventional resonance Raman spectroscopy and surface enhanced Raman spectroscopy (SERS) can be used.

Raman spectroscopy, due to the nature of its measurement method, allows a specific peak in Raman shift to shift by several cm⁻¹ per measurement. Since such peak shift per measurement is within ±10 cm⁻¹ in many cases, signals with Raman shifts within ±10 cm⁻¹ from the peak position are considered to represent the same signal.

A specific peak indicating polysulfide shifts depending on the object to be measured. For example, but without limitation, when a cancer tissue is the object to be measured, the measurement results in appearance of a peak at a Raman shift of 480 ± 10 cm⁻¹, while when a solution is the object to be measured, the measurement results in appearance of a peak at a Raman shift of 460 ± 10 cm⁻¹.

Here, the level of polysulfide when Raman spectroscopy is used represents the height of a specific peak that indicates polysulfide. When other measurement methods are used, it may represent measured values of polysulfide in cancer tissues, correction values obtained by correcting the measured values with control measurements or other values, or index values.

The anticancer drug is not particularly limited as long as it is reactive to polysulfide. Reaction of an anticancer drug with polysulfide results in elimination of the anticancer effect of the anticancer drug, so that the therapeutic effect of the anticancer drug is lost. Examples of the anticancer drug that is reactive to polysulfide include, for example, cisplatin and gemcitabine, and cisplatin is preferable.

The target tissue refers to a tissue to be measured that is collected from a patient. When the control tissue is a cancer tissue, the cancer tissue is, for example, but not limited to, an ovarian cancer tissue or a pancreas cancer tissue. Preferably, the cancer tissue is an ovarian cancer tissue. Preferably, the cancer tissue is isolated.

The resistance to an anticancer drug refers to not only the case where the effect of the anticancer drug is not obtained at all, but also, for example, the cases where the therapeutic effect is insufficiently obtained because the therapeutic effect of the anticancer drug is weak; and where the effect of the anticancer drug is temporary, and soon thereafter a relapse occurs.

Data for determining the resistance to an anticancer drug may be acquired before or after the anticancer drug is administered to patients. To previously determine whether a cancer tissue of a patient has the resistance to an anticancer drug before the anticancer drug is administered to the patient, the data is previously acquired before the anticancer drug is administered to the patient.

Another aspect of the present invention is a method of acquiring prediction data on the presence of cancer cells and/or the resistance of the cancer cells to an anticancer drug,
wherein the method uses the level of polysulfide as an index,
wherein when the level of polysulfide in a target tissue is higher than that in a control tissue, prediction is made that cancer cells are present and/or that the cancer cells have resistance to the anticancer drug in the target tissue,
wherein the anticancer drug is reactive to the polysulfide.

With respect to the measurement method, the anticancer drug, the cancer tissue, and others in this aspect, the same as those described in the method of acquiring data for determining the presence of cancer cells and/or the resistance of the cancer cells to an anticancer drug as described above may be applied (similarly for other aspects described later).

When cancer cells are determined as anticancer drug resistant, administration of the anticancer drug reactive to polysulfide can be stopped and switched to a therapeutic step in which an anticancer drug that is nonreactive to polysulfide is administered.

The control tissue refers to, for example, a normal tissue that is a corresponding portion in healthy subjects, or a cancer tissue that is a corresponding portion in other cancer patients having cancer in the corresponding tissue.

The case where the level of polysulfide in a cancer tissue is higher than the level of polysulfide in a control tissue preferably means that the level of polysulfide in the cancer tissue is higher than polysulfide in the control tissue preferably by 25% or more. A statistically significant difference is preferably present, but is not necessarily required.

Another aspect of the present invention is use of polysulfide as a marker for determining the presence of cancer cells and/or the resistance of the cancer cells to an anticancer drug, wherein the anticancer drug is reactive to the polysulfide.

The determination marker of the present invention may be used in combination with other determination markers.

Another aspect of the present invention is a kit for determining the presence of cancer cells and/or the resistance of the cancer cells to an anticancer drug, the kit comprising a substrate for measuring the level of polysulfide by Raman spectroscopy, wherein the anticancer drug is reactive to the polysulfide.

The kit may comprise, for example, a reagent and an apparatus for collecting a cancer tissue, and a substrate and an apparatus for measuring the level of polysulfide in a cancer tissue by Raman spectroscopy. The substrate is not particularly limited as long as it can be used for measurement of the level of polysulfide in Raman spectroscopy, and may be, for example, a GNF (Gold-nanofeve) substrate. The GNF substrate is a substrate in which horse bean-shaped Au nanoparticles (Gold-nanofeve (GNF)) are randomly arranged.

The method of preparing a GNF substrate is not particularly limited, and for example, the preparation can be made by the following method.

First, aluminum (Al) is deposited on a glass plate. The deposition can be done using a deposition method or a sputtering method that are known to those skilled in the art, and a sputtering method is preferable from the viewpoint of enhancing the adhesion of Al to the plate. The sputtering method can be performed using, for example, a reactive DC magnetron sputtering system (SPF-530H, ANELVA). Preferably, Al is deposited to the plate at a deposition rate of 4 to 6 Å s⁻¹. Preferably, the thickness of deposited Al is from 40 to 60 nm. Preferably, the glass plate is previously washed with a surfactant, rinsed with an ultra-purified water in a sonication bath, and dried before deposition of Al for preventing contamination of organic matter.

Next, the Al-deposited plate is boiled in hot water (100°C) preferably for 10 to 20 minutes to form boehmite (AIO(OH)) on the plate. Thereafter, the plate may be dried under nitrogen gas.

Then, gold (Au) can be deposited to the plate by a method known to those skilled in the art preferably at a deposition angle of 75 to 85° to prepare a GNF substrate. The deposition of Au can be carried out, for example, with an electron-beam evaporation system (EBX-8C, ULVAC).

A tissue section is mounted on the GNF substrate prepared as described above or other substrates, which is then irradiated with a near-infrared laserbeam to generate a near-field light. The near-field light is used to enhance the Raman scattering light that is reflective of the interatomic vibration in polysulfide. The enhanced Raman scattering light is detected with an inverted Raman microscope system (RAMANforce, Nanophoton Corporation, Suita, Osaka) to measure the level of the polysulfide.

The kit may further comprise attached documents describing the protocols and the diagnostic criteria.

### EXAMPLES

The following examples are described for the purpose of disclosure of the present invention, and are not intended to limit the scope of the present invention.

### <Materials and Methods>

### Samples Used

The experimental data was obtained using cancer tissues described in the examples with the approval of the Internal Review Boards on ethical issues of the National Defense Medical College (NDMC, Tokorozawa) and the National Cancer Center (NCC, Tokyo). The ovarian cancer tissues derived from patients that were used were obtained from NCC Biobank.

### Gel Shift Assay

To quantify the degree of DNA intercalation by cisplatin (CDDP) that disturbs DNA supercoiling, 1 µg of plasmid (pcDNA3.1 empty vector, #V79020, Invitrogen) was mixed with a predetermined concentration of Na₂Sn (n = an integer of 1 to 4), and preincubated in 10 mM phosphate-buffered saline (PBS, pH6.0) at room temperature for 5 minutes. Then, a CDDP solution was added to the solution to obtain a final concentration of 60 µM. Thereafter, the mixed solution was incubated at 37°C for 60 minutes. After the incubation, the reaction products in the mixed solution were separated by electrophoresis using 1% agarose gel for determination of the DNA secondary structure.

### Conventional Resonance Raman Spectroscopy

The conventional resonance Raman spectroscopy was carried out using a method known by those skilled in the art. As a specific example, a method described in Non-patent Document 3 can be used.

### Surface Enhanced Raman Spectroscopy (SERS)

### <Preparation of Substrate>

A 24 × 24 × 0.5 mm³ glass plate was washed with a surfactant (W304/LB93, ADEKA Corp.) to prevent contamination of organic matter. Then, the plate was rinsed with ultra-purified water in a sonication bath. After drying the plate with a spin dryer, aluminum (Al) was deposited on the plate to a thickness of 50 nm at a deposition rate of 5 Å s⁻¹ using a reactive DC magnetron sputtering system (SPF-530H, ANELVA). Thereafter, the Al film was boiled in hot water (100°C) for 15 minutes to form boehmite (AIO(OH)), and then dried under nitrogen gas. In addition, gold (Au) was deposited at a deposition angle of 80° using an electron-beam evaporation system (EBX-8C, ULVAC). Thus, a horse bean-shaped random array of Au nanoparticles named Gold-nanofeve (GNF) was assembled on the substrate. Use of the substrate in surface enhanced Raman spectroscopy (SERS) results in generation of electromagnetic hotspots as excitation sources, enabling large-area visualization of molecular vibration of metabolites in tissue sections with sufficient sensitivity and uniformity.

### <Measurement of Raman Spectrum>

The Raman spectrum was measured using an inverted Raman microscope system (RAMANforce, Nanophoton Corporation, Suita, Osaka) with a 50 × (NA = 0.65) objective lens. The microscope system comprises a x-y scanning stage, and a 24 mW 785 nm diode laser for line-scanning laser confocal system. The sensitivity and frequency of the microscope system were calibrated by the Raman shift of 520 cm⁻¹ of silicon phonon mode before SERS measurements.

### <GNF-SERS Imaging>

A metabolite in an air-dried tissue section prepared from a frozen block of a pancreas cancer was visualized by mounting a 5 µm-thickness tissue section on a GNF substrate, and kept the substrate in a vacuum drying chamber.

Before the SERS imaging experiment, three microscratches were formed on the external region of the tissue slice on the surface of the SERS substrate using a microneedle. These microscratches are identifiable by light microscopy and SERS imaging, and thus were useful to match the orientations of SERS imaging and HE staining. To achieve a SERS imaging at a higher S/N ratio, SERS signals were accumulated at a central peak wavenumber ±10 cm⁻¹.

### <Pathological Annotation of Cancerous Portion and Cancer Stromal Portion>

Immediately after the SERS imaging of tissue sections, HE staining was performed on the same tissue sections. The microscopic images of the HE stained tissue sections mounted on an optically transparent GNF substrate were imported as digital photo files by using NanoZoomer v.2.0-HT (Hamamatsu Photonics). Cancer cells were annotated as cancerous portion or cancer stromal portion by a professional pathologist using NDP view 2 software (Hamamatsu Photonics). In the case where cancer cells were contained in a possible region of stromal portion in a cancer tissue, the region was not annotated.

### <Example 1: Identification of Protein Expressed in CCC (Ovarian Clear Cell Carcinoma)>

The present inventors had found that cancers that were insusceptible to anticancer drugs included CCC (ovarian clear cell carcinoma), while cancers that were susceptible to anticancer drugs included SC (ovarian serous carcinoma).

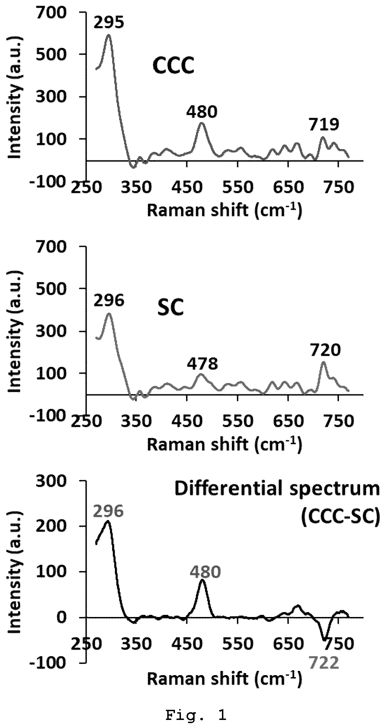

Surface enhanced Raman spectroscopy (SERS) was performed to examine whether different proteins were expressed between CCC and SC. To determine the number of peaks in SERS spectrum specific to cancerous regions of CCC, the spectra were measured in 12 CCC patients and 12 SC patients as controls, and then averaged (FIG. 1). Further, a spectrum diagram was prepared representing the difference between the CCC and SC spectra by subtracting the average SC spectrum from the average CCC spectrum. As the results, the CCC-dominant peaks appeared at Raman shifts of 295 to 296 cm⁻¹ and 478 to 480 cm⁻¹, while the SC-dominant peaks appeared at 720 to 722 cm⁻¹. Since these peaks were modestly broad, the range of each peak ± 10 cm⁻¹ was considered as a single signal for analysis.

From the previous results by the present inventors and others, it has been demonstrated that the peaks at 298 cm⁻¹, 480 cm⁻¹, and 978 cm⁻¹ in the SERS spectrum represent the presence of reactive sulfur species, such as reduced glutathione (GSH), polysulfide, and hypotaurine, respectively (Non-patent Document 1). Thus, the peak at 480 cm⁻¹ found in the SERS spectrum showing the difference between CCC and SC was identified as polysulfide.

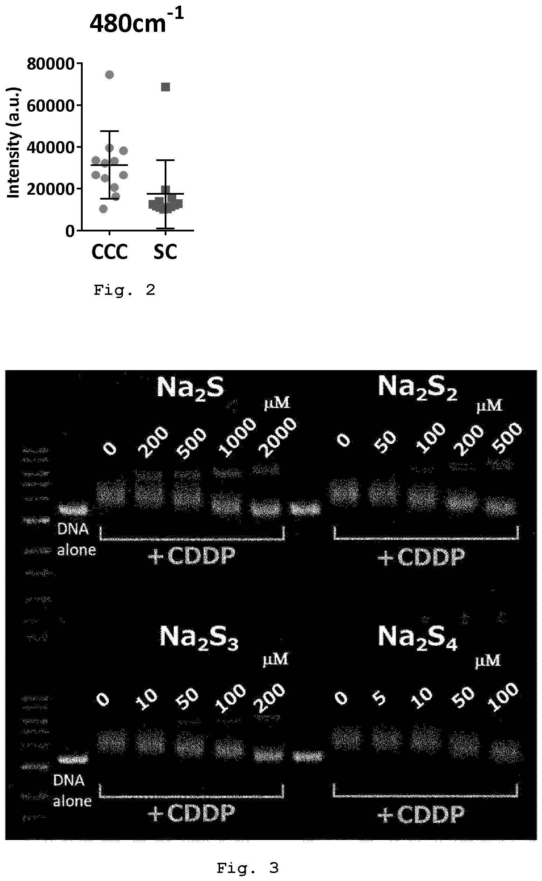

In addition, the statistical significance in the difference between CCC and SC was investigated, and it was found that the peak at 480 cm⁻¹ indicating the presence of polysulfide showed significant difference between CCC and SC (FIG. 2). However, no statistically significant difference for the peaks at 298 cm⁻¹ and 978 cm⁻¹ was found between CCC and SC.

### <Example 2: Disturbance of Double Strand DNA Supercoiling by Cisplatin (CDDP), and Canceling Effect on Supercoiling Disturbance by Polysulfide>

It was previously shown that cisplatin can disturb DNA supercoiling by being intercalated between bases of double-stranded DNA structures and causing structural changes (Non-patent Document 4). In this study, the present inventors determined in vitro that cisplatin induced change in the bulk molecular volume of supercoiling DNA, and the bulk molecular volume was recovered by addition of polysulfide (FIG. 3).

First, cisplatin increased the bulk molecular volume of double strand DNA. This suggests that cisplatin disturbs DNA supercoiling. It was demonstrated that the stepwisely increasing concentrations of polysulfide repressed the cisplatin-induced increase in the volume of the DNA molecule depending on the concentration (FIG. 3). Remarkably, it was found that the increasing number of sulfur atoms in the polysulfide resulted in increased repressing effects of the cisplatin-induced increase in the volume of the DNA molecule. This can be inferred because of the enhanced reducing power of the polysulfide with the increase in the number of sulfur atoms.

### <Example 3: Determination of Disappearance of Conventional Resonance Raman Spectroscopic Signal from Polysulfide Due to Cisplatin>

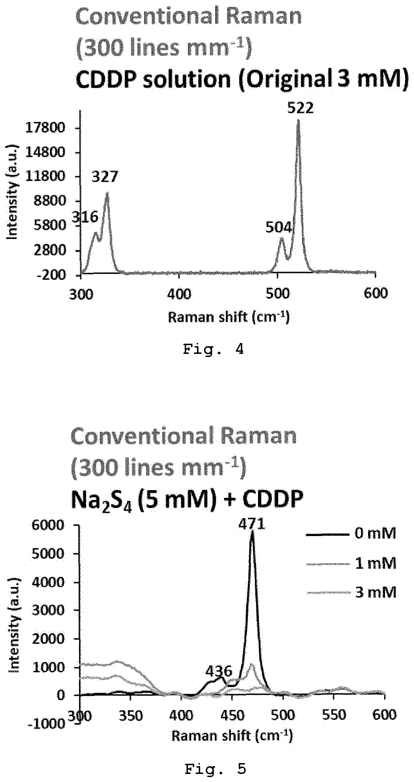

To determine whether cisplatin interacts directly with polysulfide such as Na₂S₄, the spectra from non-SERS conventional resonance Raman spectroscopy were investigated (FIGS. 4 and 5). As can be seen from the figures, cisplatin is characterized by its four major peaks (316, 327, 504, and 522 cm⁻¹) in conventional resonance Raman spectroscopy (FIG. 4). Further, it was found that addition of 5 mM Na₂S₄ to cisplatin resulted in disappearance of these four peaks yielded by cisplatin, as well as disappearance of the peak (471 cm⁻¹) yielded by Na₂S₄ (FIG. 5). These results showed that binding of CDDP to polysulfide resulted in canceling of the DNA intercalation effect of CDDP.

From the above, it was suggested that from the Raman spectroscopy performed on cancer tissues, the high peak at 480 cm⁻¹ yielded by polysulfide, i.e., the high expression level of polysulfide in the cancer tissues can be used as an index to determine whether the cancers are anticancer drug resistant.

### <Example 4: Determination of Disappearance of SERS Signal from Polysulfide Due to Gemcitabine>

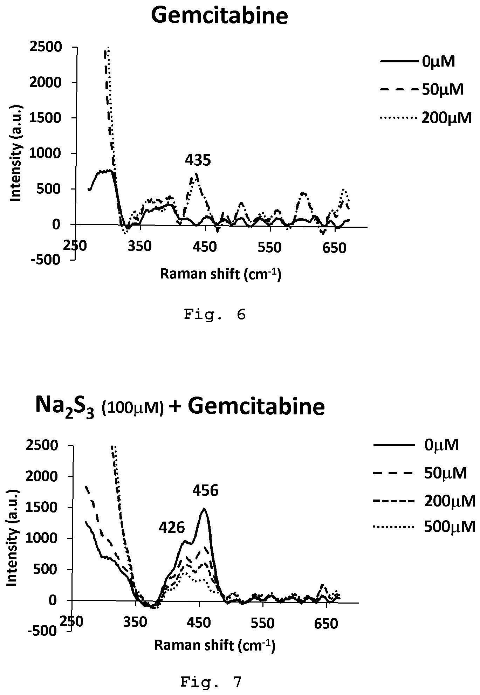

To determine whether gemcitabine interacts directly with polysulfide such as Na₂S₃ and Na₂S₄, the spectra from SERS were investigated (FIGS. 6 to 8). As can be seen from the figure (FIG. 6), gemcitabine is characterized by its one major peak (435 cm⁻¹) in the SERS spectrum. Further, it was found that addition of 100 µM Na₂S₃ or Na₂S₄ to gemcitabine resulted in disappearance of the one peak yielded by gemcitabine, as well as disappearance of the peak (456 cm⁻¹) yielded by Na₂S₃ or Na₂S₄ (FIGS. 7 and 8). These results showed that binding of gemcitabine to polysulfide resulted in disappearance of the effect as an anticancer drug of gemcitabine.

### <Example 5: Detection of Polysulfide in Pancreas Cancer Tissue by SERS Imaging>

The present inventors performed SERS imaging of pancreas cancer tissues according to the method described above and HE staining of pancreas cancer tissues according to the method known to those skilled in the art. In the SERS imaging, signals within the range of 480 ± 10 cm⁻¹ in Raman shift were considered as the same signals.

The SERS imaging demonstrated that the pancreas cancer exhibited strong SERS signals at 480 ± 10 cm⁻¹ throughout the cancer tissues including not only cancerous portions (the portions indicated by black annotation in the HE staining image), but also the surrounding regions (stromal portions, which are the portions indicated by white annotation in the HE staining) (left top and right top panels). The result of the SERS imaging on chronic pancreatitis is shown as a control, in which only weak signals were detected in chronic pancreatitis (left bottom and right bottom panels).

Based on the above results, it is suggested that the pancreas cancer also exhibited high level of polysulfide in its cancer tissue, and thus can be determined to have resistance to anticancer drugs reactive to polysulfide. Furthermore, by usual pancreas cancer tests are difficult to determine the presence of cancer because the tests are made by inserting a needle through the gastric wall and collecting pancreas tissues under endoscopic ultrasound monitoring, which often results in obtaining the surrounding CAF (cancer associated fibroblast). However, the present invention allows for clear diagnosis of cancers.

## Claims

1. A method of acquiring data for determining the presence of cancer cells and/or the resistance of the cancer cells to an anticancer drug in a target tissue, the method comprising a step of measuring the level of polysulfide in the target tissue,
wherein the anticancer drug is reactive to the polysulfide.

2. The method according to claim 1, wherein the measurement is performed before administration of the anticancer drug.

3. The method according to claim 1 or 2, wherein the measurement step is performed by detecting a peak that is specific to the polysulfide by Raman spectroscopy.

4. The method according to claim 3, wherein the specific peak appears at a Raman shift of 480 ± 10 cm⁻¹ when the Raman spectroscopy is performed on a cancer tissue.

5. The method according to any one of claims 1 to 4, wherein the anticancer drug is cisplatin or gemcitabine.

6. The method according to any one of claims 1 to 5, wherein the target tissue is an ovarian cancer tissue or a pancreas cancer tissue.

7. The method according to claim 6, wherein the ovarian cancer tissue is an ovarian clear cell carcinoma tissue.

8. A method of acquiring prediction data on the presence of cancer cells and/or the resistance of the cancer cells to an anticancer drug,
wherein the method uses the level of polysulfide as an index,
wherein when the level of polysulfide in a target tissue is higher than that in a control tissue, prediction is made that cancer cells are present and/or that the cancer cells have resistance to the anticancer drug in the target tissue,
wherein the anticancer drug is reactive to the polysulfide.

9. Use of polysulfide as a marker for determining the presence of cancer cells and/or the resistance of the cancer cells to an anticancer drug,
wherein the anticancer drug is reactive to the polysulfide.

10. A kit for determining the presence of cancer cells and/or the resistance of the cancer cells to an anticancer drug, the kit comprising a substrate for measuring the level of polysulfide by Raman spectroscopy,
wherein the anticancer drug is reactive to the polysulfide.
